(19) Europäisches Patentamt

European Patent Office

Office européen des brevets

(11) EP 1 206 942 B1

(12) EUROPEAN PATENT SPECIFICATION

(45) Date of publication and mention
of the grant of the patent:
**04.10.2006 Bulletin 2006/40**

(51) Int Cl.:
*A61K 45/00* (2006.01)  *A61K 31/22* (2006.01)
*A61K 31/4965* (2006.01)  *A61P 27/02* (2006.01)
*A61K 31/045* (2006.01)

(21) Application number: **00951907.5**

(22) Date of filing: **09.08.2000**

(86) International application number:
**PCT/JP2000/005323**

(87) International publication number:
**WO 2001/012225 (22.02.2001 Gazette 2001/08)**

(54) **WATER CHANNEL OPENER COMPOSITIONS AND MEDICINAL COMPOSITIONS FOR OPHTHALMIC USE**

ZUSAMMENSETZUNGEN ZUR ERÖFFNUNG DER WASSERKANÄLE UND MEDIZINISCHE ZUSAMMENSETZUNGEN ZUR OPTHALMISCHER VERWENDUNG

COMPOSITIONS DE SYSTEMES ASSURANT L'OUVERTURE DES CANAUX AQUEUX ET COMPOSITIONS THERAPEUTIQUES A USAGE OPHTALMIQUE

(84) Designated Contracting States:
**AT BE CH CY DE DK ES FI FR GB GR IE IT LI LU MC NL PT SE**

(30) Priority: **10.08.1999 JP 22676199**

(43) Date of publication of application:
**22.05.2002 Bulletin 2002/21**

(73) Proprietor: **SANTEN PHARMACEUTICAL CO., LTD.**
**Higashiyodogawa-ku,**
**Osaka-shi,**
**Osaka 533-8651 (JP)**

(72) Inventors:
 • **MITA, Shiro**
  **Ikoma-shi, Nara 630-0101 (JP)**
 • **ISHIDA, Naruhiro**
  **Ikoma-shi, Nara 630-0101 (JP)**
 • **HIRAI, Shin-ichiro**
  **Ikoma-shi, Nara 630-0101 (JP)**

(74) Representative: **Hart Davis, Jason et al**
 **Cabinet Beau de Loménie,**
 **158, rue de l'Université**
 **75340 Paris Cedex 07 (FR)**

(56) References cited:
 **EP-A- 0 473 159**   **EP-A- 0 917 873**
 **EP-A- 1 016 406**   **EP-A1- 0 473 159**
 **EP-A2- 0 451 130**   **JP-A- 7 126 163**
 **JP-A- 11 180 858**   **JP-A- 11 279 194**
 **US-A- 5 698 533**

 • **DATABASE WPI Section Ch, Week 199732 Derwent Publications Ltd., London, GB; Class B05, AN 1997-347386 XP002271258 & JP 09 143064 A (TAISHO PHARM CO LTD), 3 June 1997 (1997-06-03)**
 • **ISHIDA N ET AL: "IMMUNOLOCALIZATION OF AQUAPORIN HOMOLOGS IN MOUSE LACRIMAL GLANDS" BIOCHEMICAL AND BIOPHYSICAL RESEARCH COMMUNICATIONS, ACADEMIC PRESS INC. ORLANDO, FL, US, vol. 238, 1997, pages 891-895, XP002923019 ISSN: 0006-291X**
 • **PEVSNER J. ET AL.: 'Odorant-binding protein: Localization to nasal glands and secretions' PROC. NATL. ACAD. SCI. USA. vol. 83, no. 13, July 1986, pages 4942 - 4946, XP002933773 & DATABASE CAPLUS [Online] AMERICAN CHEMICAL SOCIETY (ACS) (COLUMBUS, OH, USA) Retrieved from STN Database accession no. 105:58388**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

TECHNICAL FIELD

The present invention relates to a novel water channel opener comprising a substance binding to lipocalins, particularly odorant binding proteins (hereinafter, OBP). The novel water channel opener according to the present invention has aquaporin 5 water channel-opening activity and finds application in pharmaceutical compositions, particularly as a therapeutic drug for keratoconjunctival epithelial impairment, for example a therapeutic drug for xerophthalmia (dry eye).

BACKGROUND ART

The permeation of water across the cell membrane occurs slowly by diffusion through the lipid bilayer which is a major structural element of the cell membrane. In recent years, however, a rapid movement of water across the cell membrane was discovered in certain kinds of cells, and investigations led to the postulate that, in this phenomenon, some membrane proteins which are selectively permeable to water are involved. Thereafter, a number of membrane proteins of this kind were actually isolated and these membrane proteins have come to be called water channels.

As such water channel proteins, a group of membrane proteins called aquaporins (AQP) have been isolated, and to this day such aquaporins as AQP1 to 5, FA-CHIP, and AQP-γTIP have beer discovered in mammals, amphibians and plants (e.g. Advances in Medicine, 173, 9, 745-748 (1995)). Among these, AQP5 is known to exist in the salivary gland, eye (lacrimal gland, corneal epithelial tissue), and bronchus of mammals (Advances inMedicine, 173, 9, 745-748 (1995); Am. J. Physiol., 270, C12-C30 (1996)).

AQP5 has recently been found to exist in the apical membrane of the lacrimal gland tissue cell of the eye (Ishida et al., Biochem. Biophys. Res. Comn., 224, 1-4 (1996)) and is confirmed to be deeply associated with the intercellular transport of water in lacrimation and modulating the release of tear fluid (Ishida et. al., Biochem. Biophys. Res. Comn., 238, 891-895 (1997)).

According to the present inventor's recent research, an experiment using oocytes of Xenopus laevis in which AQP5 is expressed suggested that the gate of the water channel of aquaporin is opened and closed by a certain protein of the lacrimal gland cell origin. Then, a specific partial peptide in the vicinity of the C-terminal region of AQP5 was identified for the first time as a water channel opener.

Meanwhile, studies on OBP have been undertaken in association with the elucidation of olfaction. OBP is a soluble low-molecular-weight protein which occurs at high levels in the nasal mucus of vertebrates and the sensillum lymph of insects. OBP has affinities for odorous substances and pheromones, thus is considered to be related with olfactory perception, and is known to belong to the lipocalin super family. Lipocalin is a soluble secretory protein and several members capable of binding a variety of hydrophobic ligands inclusive of odorous substances are known.

While several kinds of OBP, according to sources, have been isolated, many are dimers of which subunit is about 20 kDa, and there also are monomers. As to the OBP of vertebrates, it is synthesized, to the best of our knowledge, in the nasal passage tissue, secreted extracellularly, and accumulated at the highest level in the nasal respiratory epithelium.

It is also known that ligands of lipocalins, particularly odorous substances having OBP-binding activity (hereinafter referred to sometimes as "odorant substances"), have certain biochemical or physiological activities. For example, it is reported that 2-ketoalkane derivatives and carvone influence sodium-potassium ATPase in the olfactory tissue (Life Sciences, 20, 1051-1062 (1977)) and that monoterpenes such as carvone inhibit lens aldose reductase (Arch. Pharm. Res., 11, 312-314 (1988)), while Japanese Kokai Publication Hei-2-193932 discloses transdermal and transmucosal absorption promoting activity of carvone and others.

Regarding OBP, U.S.P. 5, 030, 722 discloses an OBP protein derived from the rat's lateral nasal gland.

It is known that OBP also exists at high levels in the rat tear fluid (Proc. Natl. Acad. Sci. USA, 83, 4942-4946 (1986)).

It is also reported that the lacrimal prealbumin secreted from the human lacrimal gland has homology with OBP in amino acid sequence (Chem. Senses, 20, 69-76 (1995)). Furthermore, a 19kDa protein closely resembling lacrimal lipocalin has been discovered in the human nasal mucus (Comp. Biochem. Physiol., 118B, 819-824 (1997)).

A protein capable of binding to an odorous substance which is also considered to belong to the lipocalin super family has been detected in the animal urine and saliva (Finlayson et al., Science, 149, 981-982 (1965), Cell, 32, 755-761 (1983)).

EP-A-1 016 406 (SENJU PHARMA CO) 5 July 2000 (2000-07-05) & WO99/09968 4 March 1999 (04.03.1999) discloses an ophthalmic composition comprising lipocalin ligands having odorant protein-binding activity (i.e. menthol, borneol etc.). The eyedrops which contain menthol reduce dry feeling of the eye.

EP-A-0 473 159 (SENJU PHARMA CO) 4 March 1992 (1992-03-04) discloses an ophthalmic composition comprising retinol (being a lipocalin ligand having odorant protein-binding activity) and its use in the treatment of dry eyes.

US-A-5 698 533 (KANG MENG-CHE) 16 December 1997 (1997-12-16) discloses an ophthalmic composition comprising menthol (being a lipocalin ligand having odorant protein-binding activity) for the treatment of dry eyes.

## SUMMARY OF THE INVENTION

Despite the thus-gathered information on lipocalins, inclusive of OBP, and odorous substances, their relation to lacrimation has been unknown.

However, in the course of the research into the action of ligands of lipocalins inclusive of OBP in the lacrimal gland, the inventors of the present invention found surprisingly that various ligand substances including odorous substances exhibit a lacrimation stimulating activity.

In view of the above state of the art, the present invention has for its object to provide a novel water channel opener having the activity to open an AQP water channel. It is a further object to provide a pharmaceutical composition, particularly a lacrimation stimulant, comprising the opener as an active ingredient. The above stimulant can be used in the therapy of keratoconjunctival epithelial impairment, such as xerophthalmia (dry eye).

The first aspect of the present invention is an aquaporin water channel opener composition which comprises a ligand of lipocalin.

In this first aspect of the invention, the ligand of lipocalin is preferably a compound having odorant binding protein-binding activity.

The second aspect of the present invention is a pharmaceutical composition for ophthalmic use which comprises a ligand of lipocalin as an active ingredient.

The pharmaceutical composition for ophthalmic use according to the second aspect of the present invention is preferably a lacrimation stimulant composition or a therapeutic drug for keratoconjunctival epithelial impairment.

In the second aspect of the invention, the therapeutic drug for keratoconjunctival epithelial impairment is preferably a therapeutic drug for xerophthalmia.

In the second aspect of the invention, the ligand of lipocalin is preferably a compound having odorant binding protein-binding activity.

Preferably, the second aspect of the invention further comprises a parasympathomimetic drug.

## BRIEF DESCRIPTION OF THE DRAWINGS

Fig. 1 is a graph showing the results of a water permeability experiment performed with carvone in Example 1.

Fig. 2 is a graph showing the results of water permeability experiments performed with odorant substances in Example 2.

Fig. 3 is a graph showing the effect of the combined use of pilocarpine hydrochloride on mouse lacrimation outputs in Example 4.

## DETAILED DISCLOSURE OF INVENTION

The present invention is now described in detail.

The composition according to the first aspect of the present invention is an aquaporin water channel opener composition having AQP5 water channel-opening activity. As used in this specification, the term AQP5 water channel-opening activity means the potency to enhance the water permeability of the cell membrane through the AQP5 water channel and the term aquaporin water channel opener means a substance having aquaporin water channel-opening activity. The water channel mentioned above includes channels which selectively allow only water to pass and channels which allow not only water to pass but allow low-molecular-weight molecules, such as glycerol or urea, to pass.

The AQP5 water channel-opening activity of the composition according to the first aspect of the invention is now described.

It is known that, in living tissue cells, the water permeating activity by AQP5 is modulated in various ways. For example, even in the lacrimal gland tissue cells with verified expression of AQP5, the lacrimation is normally controlled by sympathetic-parasympathetic innervation. Therefore, the expression of AQP5 in a living tissue cell and the expression of its water permeating activity are not one and the same event. Therefore, the water permeating activity by AQP5 is confirmed by a water permeability experiment using oocytes of platanna (Xenopus laevis) , which is known to have none of AQP family genes expressed therein and is injected with the AQP5 gene for expression of the encoded protein, as follows.

Injection of the AQP5 mRNA into oocytes of Xenopus laevis triggers an elevation in water permeability. Since this elevation in water permeability can be easily observed, this technique has been used broadly for the confirmation of water channel activity. For example, Ishibashi et al. inserted the cDNA of AQP3 into the pSP64T-derived BlueScript vector, synthesized cRNA using T7RNA polymerase, injected this cRNA into oocytes of <u>Xenopus laevis</u>, and confirmed an enhancement of water permeability as evidenced by an increase in volume in 48 to 62 hours of incubation after injection, thus proving the existence of the water channel (Proc.Natl. Acad. Sci. USA, <u>91</u>, 6269-6273 (1994)).A similar report is also found in Science, <u>256,</u> 385-387 (1992).

Therefore, in the same manner as the above, oocytes of <u>Xenopus laevis</u> are microinjected with the full-length cRNAcoding for AQP5 and cultured for expression of AQP5. Then, the oocytes are transferred to a hypotonic culture fluid and the water permeability is calculated from the change in volume. Whereas the control group not injected with the full-length cRNA coding for AQP5 shows only low water permeability, the water permeability is elevated in the group injected with the full-length cRNA coding for AQP5, thus substantiating the water channel activity.

On the other hand, in the system where oocytes of <u>Xenopus laevis</u> are microinjected with both the full-length cRNA coding for AQP5 and the poly (A)$^+$ RNA derived from the lacrimal gland and cultured to cause expression of the AQP5 protein and the total protein of the lacrimal gland origin, the water permeating activity of AQP5 is drastically decreased compared with the above-mentioned system in which AQP5 alone is expressed as is hereinlater described in further detail in Examples. Therefore, it is clear that the water permeating activity of AQP5 is inhibited by the presence of the protein of the lacrimal gland origin. This phenomenon of the water permeating activity of AQP5 being inhibited by the presence of the protein of the lacrimal gland origin is considered to arise from changes in the opening and closing of the water channel gate.

When the composition according to the first aspect of the invention is caused to coexist in this system, the water permeating activity of AQP5 recovers to a level comparable to that observed in the absence of the inhibition. The fact that the ligand of lipocalin is identified as a substance having an AQP5 water channel opening-activity is quite a surprise in light of the knowledge so far gathered about lipocalins, particularly OBP.

Lipocalins are comparatively small soluble proteins occurring in various organs and body fluids of vertebrates and invertebrates, and they show diversity at the amino acid sequence level. However, lipocalins in the group called kernel lipocalin have 3 conserved sequence motifs in common in the vicinity of the N-terminus, and those in the group called outlier lipocalin, of which OBP is a member, have one of said sequence motifs in common. Furthermore, lipocalins have a quite distinctive common feature in the high dimension structure and the typical lipocalin structure comprises a β barrel structure consisting of 8 consecutive anti-parallel β-strands, with a $3_{10}$-like helix and an α-helix being attached to the respective ends of this hydrophobic pocket-like structure. Ligands bind to the hydrophobic β-barrel. Therefore, lipocalins have high affinities for hydrophobic molecules and are suspected to be functioning <u>in vivo</u> as carriers of hydrophobic ligands in body fluids.

As examples for the substances known as lipocalins, there can be mentioned, for example, OBP, α-1-microglobulin, α1-acid glycoprotein, apolipoprotein, crustacyanin, embryo CH21 protein, β-lactoglobulin, major urinary protein, probasins, retinol binding protein, lacrimal albumin, von Ebner gland protein, purpurin, and so forth.

It has been confirmed with bovine OBP that OBP has the typical β-barrel motif in common with lipocalins. The mouse OBP is a heterodimer comprising two subunits Ia and Ib. The nucleotide sequences of the mouse OBP-Ia and Ib genes (657bp and 669bp, respectively) and the amino acid sequences of OBP-Ia and Ib proteins (147 amino acid residues and 146 amino acid residues, respectively) are disclosed in Gene, <u>212</u>, 49-55 (1998).

OBP includes the following proteins. Thus, bovine OBP, rat OBP-I, rat OBP-II, rabbit OBP-I, rabbit OBP-II, porcine OBP-I, porcine OBP-II, mouse OBP-I, mouse OBP-II, mouse OBP-III, hys-OBP-I (porcupine), hys-OBP-II (porcupine), deer OBP-I, deer OBP-II, frog BG, feline OBP, etc. are examples of OBP which have been isolated from the olfactory tissues of vertebrates.

As the hydrophobic molecules binding as ligands to lipocalins, there can be mentioned, for example, retinol (a ligand of lacrimal albumin, retinol binding protein, purpurin, etc.), glycolipids (ligands of VEG protein), porphyrins (ligands of protein HC) , denatonium benzoate (a ligand of von Ebner gland protein) , as so forth. The ligands are described in detail below, taking OBP as an example.

The OBP takes a broader range of substances as ligands as compared with other lipocalins, and it reversibly binds various odorous substances (odorant substances) and pheromones. The existence of 2-isobutyl-3-methoxypyrazine-binding activity is one of the characteristics of OBP proteins (however, Feline OBP is the only known exception). While an odorous substance is a substance which stimulates olfactory sensation, having OBP-binding activity is not necessarily the equivalent of eliciting an olfactory sensation. Therefore, in the present invention, it does not matter whether a substance having OBP-binding activity evokes an olfactory sensation. Ligands of OBP having OBP-binding activity in general have polar groups such as hydroxyl, carbonyl, etc. or a heterocycle and are medium-sized molecules having a planar hydrophobic region. There can be mentioned, but not limited to, 2-isobutyl-3-methoxypyrazine, 2-amino-4-butyl-5-propylselenazole, citronellyl acetate, carvone, 2-isopentylpyrazine, 4-butyl-5-propylthiazole, thymol, menthol, 3,7-dimethyloctanol, 2-nonenal, linalool, retinol, benzyl benzoate, 3-membered-ring musk-like compounds, 2-methyl-3-methoxypyrazine, benzaldehyde, quinoline, 2-phenylethanol, cineol, isobutyl isovalerate, isovaleric acid, β-ionone, 2-trans-6-cis-nonadienal, geosmin, trichloroanisole, 5α-androst-16-en-3-one, pentadecalactone, dimethyl sulfide, 4-hydroxyoctanolactone, ethyl acetate, borneol, for example.

These substances generally have dissociation constants of about 0.1 to 100 μM. For example, 2-isobutyl-3-methoxypyrazine, 2-isopentylpyrazine, 4-butyl-5-propylthiazole, thymol, menthol, 3,7-dimethyloctanol, 2-nonenal, linalool, retinol, benzyl benzoate, 3-membered-ring musk-like compounds, etc. have dissociation constants of about 0.1 to 1 μM as determined with bovine OBP.

The pharmaceutical composition for ophthalmic use according to the second aspect of the present invention comprises at least one species of said ligands of lipocalin, particularly ligands of OBP as an active ingredient. As used in this specification, the term "ophthalmic use" means the use in humans or animals for the therapy of a disease of the eye or for the purpose of improving or promoting the function of the eye. Since the pharmaceutical composition of the second aspect of the invention has AQP5 channel-opening activity in lacrimal gland tissue cells, it exhibits a lacrimation stimulating activity and can be used as a lacrimation stimulant composition. Furthermore, the duration of the effect canbe prolonged by using, in combination, a parasympathomimetic drug having glandular secretomotory activity, such as pilocarpine hydrochloride.

As such pharmaceutical compositions for ophthalmic use, there can be mentioned, for example, a lacrimation stimulant composition, a therapeutic drug for keratoconjunctival epithelial impairment, a keratoconjunctival wound healing accelerator, and a keratoconjunctival epithelial cell elongation stimulant, etc. Among these, the therapeutic drug for xerophthalmia (dry eye) is important as a therapeutic drug for diseases arising from the abuse of eyes associated with the development of OA equipment.

The pharmaceutical composition for ophthalmic use according to the second aspect of the present invention can directly stimulates secretion of tear fluid from the lacrimal gland. Therefore, it is quite different in the mechanism of action from the conventional ophthalmic drugs comprising artificial tear aimed at compensating for deficiencies in tear fluid, and is not only quick acting but also improving the lacrimation. The therapeutic drug for keratoconjunctival epithelial impairment according to the second aspect of the invention has, taking advantage of the above characteristics, very advantageous properties such as long-lasting action, particularly for the therapy of xerophthalmia (dry eye).

The disease at which the therapeutic drug for keratoconjunctival epithelial impairment according to the second aspect of the invention aims is not limited to dry eye but includes, for example, Sjögren's syndrome, Stevens-Johnson's syndrome, postoperative diseases, drug-induced diseases, traumatic diseases, and exogenous diseases caused by wearing the contact lens.

However, the pharmaceutical composition according to the second aspect of the invention is not limited to the above applications but can be administered to humans or animals as a pharmaceutical composition for the treatment of diseases of the tissues and organs in which AQP5 is expressed.

The pharmaceutical composition according to the second aspect of the invention can be administered not only topically but also systemically, that is to say orally or parenterally. The dosage form includes ophthalmic drugs such as eyedrops and ophthalmic ointments, injections, tablets, capsules, and granules, for example. These dosage forms can be prepared by the established technologies. Eyedrops, for instance, can be prepared into a dosage form by formulating an isotonizing agent such as sodium chloride, concentrated glycerin or the like; a buffer such as sodium phosphate, sodium acetate or the like; a surfactant such as polyoxyethylene sorbitan monooleate, polyoxyl 40 stearate, polyoxyethylene-hydrogenated castor oil or the like; a stabilizer such as sodium citrate, sodium edetate or the like; and a preservative such as benzalkonium chloride, parabens, or the like as needed. The pH may be within the acceptable range for ophthalmic drug preparations but the range of pH 4 to 8 is preferred. Oral preparations such as tablets, capsules, granules, etc. can be prepared into a dosage form by using an excipient such as lactose, starch, crystalline cellulose, vegetable oil, or the like; a lubricant such as magnesium stearate, talc, or the like; a binder such as hydroxypropylcellulose, polyvinylpyrrolidone, or the like; a disintegrator such as carboxymethylcellulose calcium or the like; a coating agent such as hydroxypropylmethylcellulose, macrogols, silicon resin, or the like; and a gelatin film-forming agent, as needed.

The dosage of the pharmaceutical composition according to the second aspect of the invention can be appropriately selected according to symptom, age, dosage form, and the like. When the pharmaceutical composition for ophthalmic use according to the second aspect of the invention is used as eyedrops, for instance, it is sufficient to instill a preparation containing 0.001 to 3% (w/v) of the active ingredient of the second aspect of the invention once or several times daily. Oral preparations can be administered generally 1 mg to 1000 mg per day, either once or in a few divided doses.

It has been verified by intravenous administration in mice that the pharmaceutical composition according to the second aspect of the invention produces a lacrimation stimulating activity in vivo.

BEST MODE FOR CARRYING OUT THE INVENTION

The following Experimental Examples, Examples, and Drug Preparation Examples illustrate the present invention in further detail without limiting the scope of the invention.

Experimental Example 1 <u>Preparation of lacrimal gland poly (A)± RNA</u> .

The lacrimal gland was isolated from male SD rats and male BALB/c mice and using Pharmacia RNA Purification Kit (product of Pharmacia), the total RNA was isolated. Poly (A)+ RNA was purified by affinity chromatography using an oligo (dT) cellulose column in the routine manner.

Experimental Example 2 <u>Construction of cDNA coding for AQP5</u>

The rat lacrimal gland poly (A)+ RNA obtained in Experimental Example 1 was subjected to reverse transcription using an oligo (dT)$_{18}$ primer and the single-stranded cDNA obtained was used as a template for PCR. As the PCR primer, a sequence containing restriction enzyme digesting sites and permitting PCR amplification of the open reading frame of rat AQP5 cDNA was used. The above single-stranded cDNA for use as the template was amplified by PCR (94°C, 1 min, 60°C, 1 min., 72°C, 2 min. for 30 cycles). The PCR product was subcloned in plasmid BlueScript II KS (+). In this experiment, to obtain the exact cDNA, this subcloning was performed 10 times. The nucleotide sequence of the DNA was confirmed by the chain terminator method. Hereunder, this recombinant plasmid is referred to as pBlueScript II KS (+)-AQP5.
Then, the design of primers was made to amplify the AQP5 full-coding region and to include the non-translated region at 5' -side of <u>Xenopus laevis</u> β-globin gene, the existence of which has been reported in J. Biol. Chem. , 258, 7924-7927 (1983) . Using said pBlueScriptII KS (+) -AQP5 DNA as a template and 50 pmol of primer, PCR amplification was carried out (94°C, 1 min, 50°C, 1 min, 72°C, 2 min. for 30 cycles) . The PCR product was subcloned in pSP64poly (A) vector (product of Pro-Mega). This recombinant plasmid is hereunder referred to as pSP64poly(A)-AQP5.

Experimental Example 3 <u>Synthesis of RNA</u>

Using 5 μg of the EcoR1 digest of said pSP64poly (A) -AQP5 DNA and SP6RNA polymerase, an in vitro transcription was carried out in 100 μL in the presence of cap analog m$^7$G(5')ppp(5')G at 30°C for 1 hour to synthesize the complementary RNA (cRNA). Then, the plasmid DNA was digested with RNase-free DNaseI (Pharmacia Biotech), extracted with phenol/chloroform, and extracted twice with ethanol. The cRNA thus obtained was suspended in distilled water for injection into oocytes.

Experimental Example 4 <u>Preparation of oocytes and expression of the protein</u>

Oocytes were prepared in accordance with the method described in Taylor et al. (Proc. Natl. Acad. Sci. USA., 82, 6585-6589 (1985)) as follows. Mature female individuals of <u>Xenopus laevis</u> were anesthetized and oocytes (stage V to VI) were taken out and placed in Barth's buffer solution (5 mM Tris-HCl, 88mM NaCl, 1 mM KCl, 2.4 mM NaHCO$_3$, 0.33 mM Ca(NO$_3$)$_2$, 0.41 mM CaCl$_2$, 0.82 mM MgSO$_4$, penicillin + streptomycin 10 μg/mL, pH 7.2; the buffer of this formulation is hereunder referred to as "MBS") . Then, in Barth's buffer solution containing 2 mg/mL of type II collagenase but no calcium ion, the respective cells were dispersed by gentle stirring for 1 hour. These oocytes were washed thoroughly with Barth's buffer solution. The oocytes were then cultured in Barth's buffer solution at 20°C overnight and on the following day a microinjection was carried out by the following procedure.
In 50 nL of distilled water was dissolved 5 ng of the AQP5 cRNA obtained in Experimental Example 3, either alone or together with 25 ng of the lacrimal gland poly (A)+ RNA obtained in Experimental Example 1, and the solution was microinjected into the oocytes with a sterilized glass micropipet using Drummond Microinjection System (product of Drummond). In the control group, 50 nL of distilled water was microinjected. The oocytes were cultured in MBS at 20°C for 3 days with the culture fluid changed daily to cause AQP5 and the protein of the lacrimal gland origin to be expressed.
Expression of AQP5 protein was confirmed by subjecting the membrane fraction of oocytes to SDS polyacrylamide gradient gel electrophoresis and carrying out a Western blot analysis using the rabbit antiserum obtained by using the C-terminus of AQP5. Furthermore, using an immobilized oocyte specimen, the expression of AQP5 protein in the cell membrane was confirmed by the immunofluoresence technique using a fluorescence microscope.

Example 1 <u>Test on the effect of carvone on water permeability</u>

The oocytes obtained in Experimental Example 4 were incubated in 5 mM dibutyryl cAMP-containing isotonic MBS (salt concentration ca 200 mOsm) for 30 minutes. The oocytes were then transferred to isotonic MBS, 10$^{-8}$M, 10$^{-7}$M or 10$^{-6}$M carvone was microinjected into the oocytes, and the cells were cultured in isotonic MBS for.4 hours. In the control group, distilled water was microinjected. The water permeability experiment was performed according to the following protocol.

The water permeability experiment was performed in accordance with the method described in the literature (Science, 256, 385-387 (1992), and Proc. Natl. Acad. Sci. USA., 91, 6269-6273 (1994)), as follows.

The above oocytes cultured in isotonic MBS (200 mOsm) for 4 hours were transferred to hypotonic (40 mOsm) MBS and cultured therein at 20°C and, in the course, serial photographing was made using a phase contrast microscope (product of Olympus) at 10-second intervals. The volume and the change in volume were computed from the image output of an image analysis system (product of Fuji Film) . The water permeability value (Pf) was calculated from the initial gradient of $V/V_0$ against time $(d(V/V_0)/dt)$, the initial volume of the oocyte ($V_0 = 9 \times 10^{-4}$ cm$^3$), the initial area of the oocyte (S = 0.045 cm$^2$), and the molar volume of water ($V_w$ = 18 cm$^3$/mol) by means of the following equation.

$$\mathrm{Pf\ (cm/sec)} = [V_0 \times (d(V/V_0)/dt)]/[S \times V_w \times (mOsm_{in} - mOsm_{out})]$$

In the equation, V represents the volume (cm$^3$) of the oocyte after time t; $mOsm_{in}$ represents the initial salt concentration of MBS, which was 200 mOsm in this case; $mOsm_{out}$ represents the salt concentration of hypotonic MBS, which was 40 mOsm in this case.

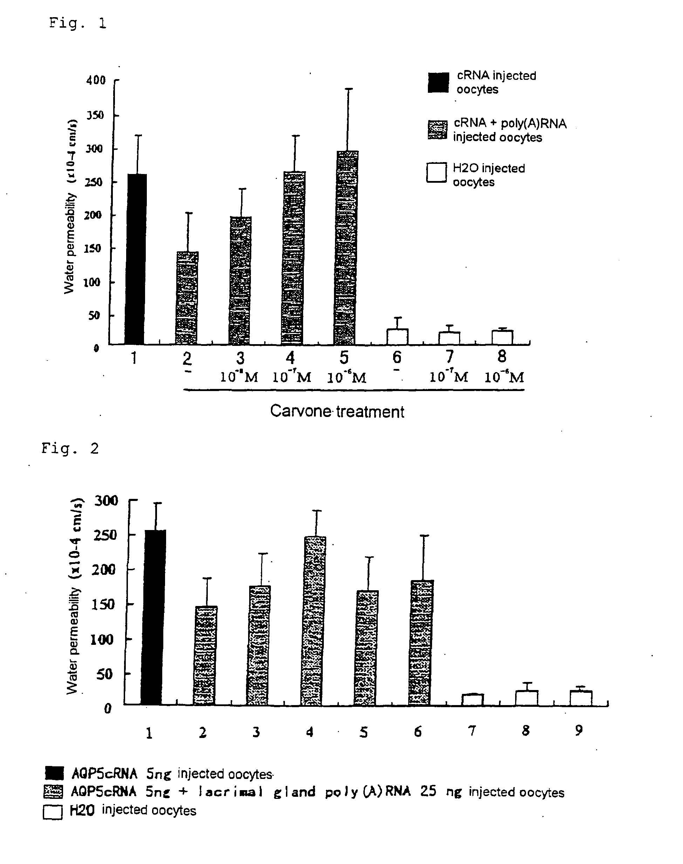

The results are shown in Fig. 1. Experiments 1 to 8 on the drawing were performed according to the following conditions 1 to 8.

|  | Oocytes | Injection |
|---|---|---|
| Experiment 1 | cRNA injected | Distilled water |
| Experiment 2 | cRNA + poly (A) RNA injected | Distilled water |
| Experiment 3 | cRNA + poly (A) RNA injected | $10^{-8}$ M Carvone |
| Experiment 4 | cRNA + poly (A) RNA injected | $10^{-7}$ M Carvone |
| Experiment 5 | cRNA + poly (A) RNA injected | $10^{-6}$ M Carvone |
| Experiment 6 | Distilled water injected | Distilled water |
| Experiment 7 | Distilled water injected | $10^{-7}$ M Carvone |
| Experiment 8 | Distilled water injected | $10^{-6}$ M Carvone |

Example 2 Test on the effect of odorant substances on water permeability

A water permeability experiment was carried out in the same manner as Example 1 using 2-isobutyl-3-methoxypyrazine, $10^{-7}$ M or $10^{-6}$ M, or citronellyl acetate, $10^{-7}$ M or $10^{-6}$ M, each as an another odorant substance, in lieu of carvone.

The results are shown in Fig. 2. Experiments 1 to 9 on the drawing were performed under the following experimental conditions 1 to 9.

|  | Oocytes | Injection |
|---|---|---|
| Experiment 1 | cRNA injected | Distilled water |
| Experiment 2 | cRNA + poly (A) RNA injected | Distilled water |
| Experiment 3 | cRNA + poly (A) RNA injected | $10^{-7}$M 2-isobutyl-3-methoxypyrazine |
| Experiment 4 | cRNA + poly (A) RNA injected | $10^{-6}$ M 2-isobutyl-3-methoxypyrazine |
| Experiment 5 | cRNA + poly (A) RNA injected | $10^{-7}$ M citronellyl acetate |
| Experiment 6 | cRNA + poly (A) RNA injected | $10^{-6}$ M citronellyl acetate |
| Experiment 7 | Distilled water injected | Distilled water |
| Experiment 8 | Distilled water injected | $10^{-6}$ M 2-isobutyl-3-methoxypyrazine |
| Experiment 9 | Distilled water injected | $10^{-6}$ M citronellyl acetate |

Referring to Example 1, Experiment 1 showed the water permeability of oocytes not injected with poly (A) $^+$ RNA of the lacrimal gland origin but expressing AQP5 protein; the result clearly indicated the existence of the activity of a water channel when compared with Experiments 6 to 8 in which AQP5 was not expressed. Experiments 2 to 5 showed the water permeability of oocytes in the presence of both AQP5 protein and the protein expressed by injection of poly (A)$^+$ RNA of the lacrimal gland origin. It was apparent from Experiment 2 that the water permeability was considerably decreased in the presence of the protein expressed by injection of poly (A)$^+$ RNA of the lacrimal gland origin. Comparison of this result with the result of Experiment 1 indicated that the protein expressed by injection of poly (A)$^+$ RNA of the lacrimal gland origin inhibited the water permeability of AQP5 protein. It was confirmed from Experiments 3 to 5 that the injection of carvone into the oocytes under the above condition resulted in the recovery of water permeability to the level observed in Experiment 1.

Referring to Example 2, Experiments 3 to 6 indicated that, though not as high as carvone, 2-isobutyl-3-methoxypyrazine or citronellyl acetate also elevated water permeability. From these results, it was found that many different substances varying much in chemical structure, such as carvone which is a cyclic hydrocarbon derivative (monoterpene ketone), 2-isobutyl-3-methoxypyrazine which is a heterocyclic compound derivative, and citronellyl acetate which is a chain hydrocarbon derivative, elevate the water permeability of AQP5 in remarkable measures.

Thus, the composition of the present invention restores and maintains the water permeating activity level of cells depressed by the protein expressed by injection of poly (A)$^+$ RNA of the lacrimal gland origin in the presence of AQP to a more active state.

Example 3 Lacrimation stimulating activity of odorant substances in mice

Then, using (R)-(-)-carvone, (+)-pregon, borneol, trans-4-methylcyclohexanol, and menthol, all of which are odorant substances, each dissolved in 0.1% hydrogenated castor oil-saline, the lacrimation-stimulating activities of these substances were evaluated in vivo. The method used was as follows.

(Determination of lacrimation output)

The tear fluid was collected from the left eye of mice at 15-min intervals using a glass microcapillary tube (aspiration capacity 0.5 $\mu$L/32 mm) . The amount of aspirated tear fluid was measured in length (mm) using calipers and converted to $\mu$L for use as a marker of lacrimation output. Mice were anesthetized by intraperitoneal administration of Nembutal Anesthetic Solution (0.2 mL/10 g body weight, dosage 60 mg/kg) . After the loss of pain reflex was confirmed in the mice, the measurement of lacrimation output was started. Measurements were invariably carried out at 15-min intervals from the start to the end. After two initial measurements made 15 minutes apart, each test substance (dosage 30 mg/kg) was immediately administered into the caudal vein of mice at a ratio of 0.1 mL/10 g body weight. Thereafter, the collection of tear fluid was carried out at 15-min intervals.

As a result, marked increases in lacrimation were observed at 15 minutes after intravenous administration of each of odorant substances. The results are shown in Table 1. The change in lacrimation output was expressed in the percentage of the lacrimation output at each measurement relative to the average of the lacrimation outputs at two initial measurements prior to administration of each test substance.

Table 1

| Test substance | Change in lacrimation output after 15 minutes (%) |
| --- | --- |
| (R)-(-)-carvone | 61.5 |
| (+)-pregon | 70.2 |
| Borneol | 97.5 |
| Trans-4-methylcyclohexanol | 85.4 |
| Menthol | 88.1 |
| Vehicle | 2.8 |

Example 4 Effect of the combined use of an odorant substance and pilocarpine hydrochloride on the lacrimation output in mice

The effect of the combined use of (R)-(-)carvone, i.e. the odorant substance used in Example 1, and pillocarpine hydrochloride was evaluated. The method used was as follows.

(Determination of lacrimation output)

The tear fluid was collected from the left eye of mice at 15-min intervals using a glass microcapillary tube (aspiration capacity 0.5 $\mu$L/32 mm). The amount of aspirated tear fluid was measured in length (mm) using calipers and converted to $\mu$L for use as a marker of lacrimation output. Mice were anesthetized by intraperitoneal administration of Nembutal Anesthetic Solution (0.2 mL/10 g body weight, dosage 60 mg/kg). After the loss of pain reflex was confirmed in the mice, the measurement of lacrimation output was started. Measurements were invariably carried out at 15-min intervals from the start to the end. After two initial measurements made 15 minutes apart, the test substance was immediately administered into the caudal vein of mice at a ratio of 0.1 mL/10 g body weight. Then, the measurement was repeated at 15-min intervals. Immediately after the measurement of lacrimation output at 15 minutes following administration of the test substance, 0.005% pilocarpine hydrochloride was administered subcutaneously at 0.1 mL/10 g body weight (dosage 5 mg/kg). The collection of tear fluid was further carried out 4 times at 15-min intervals.

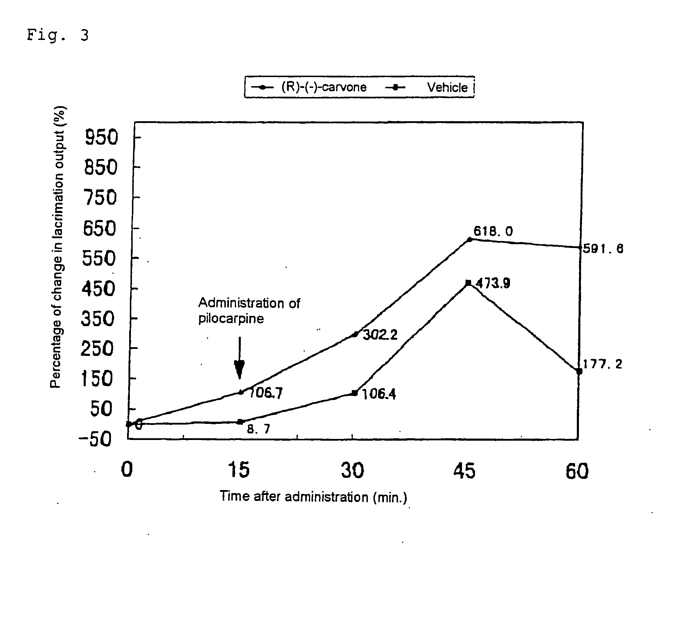

As a result, marked increases in lacrimation were noted in 10 and odd minutes to tens of minutes after intravenous administration and the effect was sustained for a long time. The results on (R)-(-)-carvone (dosage 30 mg/kg) are shown in the graph in Fig. 3. The change in lacrimation output was expressed in the percentage of the lacrimation output at each measurement relative to the average of the lacrimation outputs at the two measurements prior to administration of the test substance. The dimension of time represents the time after administration of carvone.

It couldbe observed from Example 3 that the administration of carvone causes an increase of not less than 60% in lacrimation output in a short time such as 15 to 30 minutes. Moreover, the effect diminished gradually with time. However, Example 4 showed that the combined use of pilocarpine resulted in a further prolongation of the effect.

Drug Preparation Examples

Some general formulation examples for eyedrops are given below but these are intended to assist in the understanding of the present invention and are by no means defining the scope of the invention.

| Eyedrop 1 (in 100 mL) | |
| --- | --- |
| Carvone | 100 mg |
| 1% Hydrogenated castor oil | 1 mL |
| Physiological saline | q.s. |

| Eyedrop 2 (in 100 mL) | |
| --- | --- |
| Denatonium benzoate | 100 mg |
| 1% Hydrogenated castor oil | 1 mL |
| Physiological saline | q.s. |

| Eyedrop 3 (in 100 mL) | |
| --- | --- |
| Carvone | 10 mg |
| Concentrated glycerin | 2500 mg |
| Polysorbate 80 | 2000 mg |
| Sodium dihydrogen phosphate·$2H_2O$ | 200 mg |
| 1N-Sodium hydroxide | q.s. |
| 1N-Hydrochloric acid | q.s. |
| Sterile purified water | q.s. |

Eyedrops containing carvone in concentrations of 0.001%, 0.005%, 0.05%, 0.1% and 3.0% (w/v) can also be similarly prepared by adjusting the formulating levels of carvone and additives properly.

| Eyedrop 4 (in 100 mL) | |
| --- | --- |
| 2-Isobutyl-3-methoxypyrazine | 10 mg |
| Concentrated glycerin | 2500 mg |
| Polysorbate 80 | 2000 mg |
| Sodium dihydrogen phosphate·$2H_2O$ | 200 mg |
| 1N-Sodium hydroxide | q.s. |
| 1N-Hydrochloric acid | q.s. |
| Sterile purified water | q.s. |

| Eyedrop 5 (in 100 mL) | |
| --- | --- |
| Citronellyl acetate | 10 mg |
| Concentrated glycerin | 2500 mg |
| Polysorbate 80 | 2000 mg |

(continued)

| Eyedrop 5 (in 100 mL) | |
|---|---|
| Sodium dihydrogen phosphate·2H$_2$O | 200 mg |
| 1N-Sodium hydroxide | q.s. |
| 1N-Hydrochloric acid | q.s. |
| Sterile purified water | q.s. |

## INDUSTRIAL APPLICABILITY

The novel water channel opener composition according to the first aspect of the invention, constituted as above, has AQP5 water channel-opening activity. The pharmaceutical composition according to the second aspect of the invention is capable of stimulating and maintaining the water channel activity of AQP5 in the lacrimal gland, and by using it there canbe provided a pharmaceutical composition for ophthalmic use, particularly a lacrimation stimulant composition having both quick-acting and long-lasting properties and a therapeutic drug for keratoconjunctival epithelial impairment.

## Claims

1. Use of a ligand of lipocalin for the manufacture of a composition for treatment of keratoconjuctival epithelial impairment, wherein
said ligand of lipocalin is a compound selected from the group consisting of: glycolipids, porphyrins, denatonium benzoate, 2-isobutyl-3-methoxypyrazine, 2-amino-4-butyl-5-propylselenazole, citronellyl acetate, carvone, 2-isopentylpyrazine, 4-butyl-5-propylthiazole, thymol, 3,7-dimethyloctanol, 2-nonenal, linalool, benzyl benzoate, 3-membered-ring musk-like compounds, 2-methyl-3-methoxypyrazine, benzaldehyde, quinoline, 2-phenylethanol, cineol, isobutyl isovalerate, isovaleric acid, β-ionone, 2-trans-6-cis-nonadienal, geosmin, trichloroanisole, 5-α-androst-16-en-3-one, pentadecalactone, dimethyl sulfide, 4-hydroxyoctanolactone, ethyl acetate, (+)-pregon and trans-4-methylcyclohexanol.

2. Use of a ligand of lipocalin for the manufacture of a composition for acceleration of keratoconjuctival wound helaing, wherein
said ligand of lipocalin is a compound selected from the group consisting of: glycolipids, porphyrins, denatonium benzoate, 2-isobutyl-3-methoxypyrazine, 2-amino-4-butyl-5-propylselenazole, citronellyl acetate, carvone, 2-isopentylpyrazine, 4-butyl-5-propylthiazole, thymol, 3,7-dimethyloctanol, 2-nonenal, linalool, benzyl benzoate, 3-membered-ring musk-like compounds, 2-methyl-3-methoxypyrazine, benzaldehyde, quinoline, 2-phenylethanol, cineol, isobutyl isovalerate, isovaleric acid, β-ionone, 2-trans-6-cis-nonadienal, geosmin, trichloroanisole, 5-α-androst-16-en-3-one, pentadecalactone, dimethyl sulfide, 4-hydroxyoctanolactone, ethyl acetate, (+)-pregon and trans-4-methylcyclohexanol.

3. Use of a ligand of lipocalin for the manufacture of a composition for treatment of keratoconjuctival epithelial cell elongation, wherein
said ligand of lipocalin is a compound selected from the group consisting of: glycolipids, porphyrins, denatonium benzoate, 2-isobutyl-3-methoxypyrazine, 2-amino-4-butyl-5-propylselenazole, citronellyl acetate, carvone, 2-isopentylpyrazine, 4-butyl-5-propylthiazole, thymol, 3,7-dimethyloctanol, 2-nonenal, linalool, benzyl benzoate, 3-membered-ring musk-like compounds, 2-methyl-3-methoxypyrazine, benzaldehyde, quinoline, 2-phenylethanol, cineol, isobutyl isovalerate, isovaleric acid, β-ionone, 2-trans-6-cis-nonadienal, geosmin, trichloroanisole, 5-α-androst-16-en-3-one, pentadecalactone, dimethyl sulfide, 4-hydroxyoctanolactone, ethyl acetate, (+)-pregon and trans-4-methylcyclohexanol.

4. Use of a ligand of lipocalin for the manufacture of a composition for treatment of xerophthalmia, wherein
said ligand of lipocalin is a compound selected from the group consisting of: glycolipids, porphyrins, denatonium benzoate, 2-isobutyl-3-methoxypyrazine, 2-amino-4-butyl-5-propylselenazole, citronellyl acetate, carvone, 2-isopentylpyrazine, 4-butyl-5-propylthiazole, thymol, 3,7-dimethyloctanol, 2-nonenal, linalool, benzyl benzoate, 3-membered-ring musk-like compounds, 2-methyl-3-methoxypyrazine, benzaldehyde, quinoline, 2-phenylethanol, cineol, isobutyl isovalerate, isovaleric acid, β-ionone, 2-trans-6-cis-nonadienal, geosmin, trichloroanisole, 5-α-androst-16-en-3-one, pentadecalactone, dimethyl sulfide, 4-hydroxyoctanolactone, ethyl acetate, (+)-pregon and trans-4-methylcyclohexanol.

**5.** A use according to any one of claims 1 to 4, wherein said ligand of lipocalin is used in combination with a parasympathomimetic drug.

**Patentansprüche**

**1.** Verwendung eines Lipocalin-Liganden für die Herstellung einer Zusammensetzung zur Behandlung einer Beeinträchtigung des keratokonjunktiven Epithels,
wobei
dieser Lipocalin-Ligand eine Verbindung ist, die ausgewählt ist aus der Gruppe bestehend aus: Glykolipiden, Porphyrinen, Denatoniumbenzoat, 2-Isobutyl-3-methoxypyrazin, 2-Amino-4-butyl-5-propylselenazol, Citronellylacetat, Carvon, 2-Isopentylpyrazin, 4-Butyl-5-propylthiazol, Thymol, 3,7-Dimethyloctanol, 2-Nonenal, Linalool, Benzylbenzoat, moschusartige Dreiring-Verbindungen, 2-Methyl-3-methoxypyrazin, Benzaldehyd, Chinolin, 2-Phenylethanol, Cineol, Isobutylisovaleriat, Isovaleriansäure, $\beta$-Ionon, 2-trans-6-cis-Nonadienal, Geosmin, Trichloranisol, 5-$\alpha$-Androst-16-en-3-on, Pentadecalacton, Dimethylsulfid, 4-Hydroxyoctanolacton, Ethylacetat, (+)-Pregon und trans-4-Methylcyclohexanol.

**2.** Verwendung eines Lipocalin-Liganden für die Herstellung einer Zusammensetzung zur Beschleunigung der keratokonjunktiven Wundheilung, wobei
dieser Lipocalin-Ligand eine Verbindung ist, die ausgewählt ist aus der Gruppe bestehend aus: Glykolipiden, Porphyrinen, Denatoniumbenzoat, 2-Isobutyl-3-methoxypyrazin, 2-Amino-4-butyl-5-propylselenazol, Citronellylacetat, Carvon, 2-Isopentylpyrazin, 4-Butyl-5-propylthiazol, Thymol, 3,7-Dimethyloctanol, 2-Nonenal, Linalool, Benzylbenzoat, moschusartige Dreiring-Verbindungen, 2-Methyl-3-methoxypyrazin, Benzaldehyd, Chinolin, 2-Phenylethanol, Cineol, Isobutylisovaleriat, Isovaleriansäure, $\beta$-Ionon, 2-trans-6-cis-Nonadienal, Geosmin, Trichloranisol, 5-$\alpha$-Androst-16-en-3-on, Pentadecalacton, Dimethylsulfid, 4-Hydroxyoctanolacton, Ethylacetat, (+)-Pregon und trans-4-Methylcyclohexanol.

**3.** Verwendung eines Lipocalin-Liganden für die Herstellung einer Zusammensetzung zur Behandlung der Verlängerung von keratokonjunktiven Epithelzellen,
wobei
dieser Lipocalin-Ligand eine Verbindung ist, die ausgewählt ist aus der Gruppe bestehend aus: Glykolipiden, Porphyrinen, Denatoniumbenzoat, 2-Isobutyl-3-methoxypyrazin, 2-Amino-4-butyl-5-propylselenazol, Citronellylacetat, Carvon, 2-Isopentylpyrazin, 4-Butyl-5-propylthiazol, Thymol, 3,7-Dimethyloctanol, 2-Nonenal, Linalool, Benzylbenzoat, moschusartige Dreiring-Verbindungen, 2-Methyl-3-methoxypyrazin, Benzaldehyd, Chinolin, 2-Phenylethanol, Cineol, Isobutylisovaleriat, Isovaleriansäure, $\beta$-Ionon, 2-trans-6-cis-Nonadienal, Geosmin, Trichloranisol, 5-$\alpha$-Androst-16-en-3-on, Pentadecalacton, Dimethylsulfid, 4-Hydroxyoctanolacton, Ethylacetat, (+)-Pregon und trans-4-Methylcyclohexanol.

**4.** Verwendung eines Lipocalin-Liganden für die Herstellung einer Zusammensetzung zur Behandlung von Xerophthalmie, wobei
dieser Lipocalin-Ligand eine Verbindung ist, die ausgewählt ist aus der Gruppe bestehend aus: Glykolipiden, Porphyrinen, Denatoniumbenzoat, 2-Isobutyl-3-methoxypyrazin, 2-Amino-4-butyl-5-propylselenazol, Citronellylacetat, Carvon, 2-Isopentylpyrazin, 4-Butyl-5-propylthiazol, Thymol, 3,7-Dimethyloctanol, 2-Nonenal, Linalool, Benzylbenzoat, moschusartige Dreiring-Verbindungen, 2-Methyl-3-methoxypyrazin, Benzaldehyd, Chinolin, 2-Phenylethanol, Cineol, Isobutylisovaleriat, Isovaleriansäure, $\beta$-Ionon, 2-trans-6-cis-Nonadienal, Geosmin, Trichloranisol, 5-$\alpha$-Androst-16-en-3-on, Pentadecalacton, Dimethylsulfid, 4-Hydroxyoctanolacton, Ethylacetat, (+)-Pregon und trans-4-Methylcyclohexanol.

**5.** Die Verwendung gemäß einem der Ansprüche 1 bis 4, wobei dieser Lipocalin-Ligand in Kombination mit einem parasympathomimetischen Arzneimittel verwendet wird.

**Revendications**

**1.** Utilisation d'un ligand de la lipocaline pour la fabrication d'une composition pour le traitement d'une déficience de l'épithélium kérato-conjonctival, dans laquelle
ledit ligand de la lipocaline est un composé choisi dans le groupe constitué par : les glycolipides, les porphyrines, le benzoate de dénatonium, la 2-isobutyl-3-méthoxypyrazine, le 2-amino-4-butyl-5-propylsélénazole, l'acétate de citronellyle, la carvone, la 2-isopentylpyrazine, le 4-butyl-5-propylthiazole, le thymol, le 3,7-diméthyloctanol, le 2-nonénal, le linalool, le benzoate de benzyle, les composés du type musc à cycle à trois côtés, la 2-méthyl-3-méthoxypyrazine, le benzaldéhyde, la quinoléine, le 2-phényléthanol, le cinéol, l'isovalérate d'isobutyle, l'acide isovalérique, la β-ionone, le 2-trans-6-cis-nonadiénal, la géosmine, le trichloroanisole, la 5α-androst-16-én-3-one, la pentadécalactone, le sulfure de diméthyle, la 4-hydroxyoctanolactone, l'acétate d'éthyle, la (+)-prégone et le trans-4-méthylcyclohexanol.

2. Utilisation d'un ligand de la lipocaline pour la fabrication d'une composition pour l'accélération de la cicatrisation d'une plaie kérato-conjonctivale, dans laquelle
ledit ligand de la lipocaline est un composé choisi dans le groupe constitué par : les glycolipides, les porphyrines, le benzoate de dénatonium, la 2-isobutyl-3-méthoxypyrazine, le 2-amino-4-butyl-5-propylsélénazole, l'acétate de citronellyle, la carvone, la 2-isopentylpyrazine, le 4-butyl-5-propylthiazole, le thymol, le 3,7-diméthyloctanol, le 2-nonénal, le linalool, le benzoate de benzyle, les composés du type musc à cycle à trois côtés, la 2-méthyl-3-méthoxypyrazine, le benzaldéhyde, la quinoléine, le 2-phényléthanol, le cinéol, l'isovalérate d'isobutyle, l'acide isovalérique, la β-ionone, le 2-trans-6-cis-nonadiénal, la géosmine, le trichloroanisole, la 5α-androst-16-én-3-one, la pentadécalactone, le sulfure de diméthyle, la 4-hydroxyoctanolactone, l'acétate d'éthyle, la (+)-prégone et le trans-4-méthylcyclohexanol.

3. Utilisation d'un ligand de la lipocaline pour la fabrication d'une composition pour le traitement d'allongement des cellules de l'épithélium kérato-conjonctival, dans laquelle
ledit ligand de la lipocaline est un composé choisi dans le groupe constitué par : les glycolipides, les porphyrines, le benzoate de dénatonium, la 2-isobutyl-3-méthoxypyrazine, le 2-amino-4-butyl-5-propylsélénazole, l'acétate de citronellyle, la carvone, la 2-isopentylpyrazine, le 4-butyl-5-propylthiazole, le thymol, le 3,7-diméthyloctanol, le 2-nonénal, le linalool, le benzoate de benzyle, les composés du type musc à cycle à trois côtés, la 2-méthyl-3-méthoxypyrazine, le benzaldéhyde, la quinoléine, le 2-phényléthanol, le cinéol, l'isovalérate d'isobutyle, l'acide isovalérique, la β-ionone, le 2-trans-6-cis-nonadiénal, la géosmine, le trichloroanisole, la 5α-androst-16-én-3-one, la pentadécalactone, le sulfure de diméthyle, la 4-hydroxyoctanolactone, l'acétate d'éthyle, la (+)-prégone et le trans-4-méthylcyclohexanol.

4. Utilisation d'un ligand de la lipocaline pour la fabrication d'une composition pour le traitement d'une xérophtalmie, dans laquelle
ledit ligand de la lipocaline est un composé choisi dans le groupe constitué par : les glycolipides, les porphyrines, le benzoate de dénatonium, la 2-isobutyl-3-méthoxypyrazine, le 2-amino-4-butyl-5-propylsélénazole, l'acétate de citronellyle, la carvone, la 2-isopentylpyrazine, le 4-butyl-5-propylthiazole, le thymol, le 3,7-diméthyloctanol, le 2-nonénal, le linalool, le benzoate de benzyle, les composés du type musc à cycle à trois côtés, la 2-méthyl-3-méthoxypyrazine, le benzaldéhyde, la quinoléine, le 2-phényléthanol, le cinéol, l'isovalérate d'isobutyle, l'acide isovalérique, la β-ionone, le 2-trans-6-cis-nonadiénal, la géosmine, le trichloroanisole, la 5α-androst-16-én-3-one, la pentadécalactone, le sulfure de diméthyle, la 4-hydroxyoctanolactone, l'acétate d'éthyle, la (+)-prégone et le trans-4-méthylcyclohexanol.

5. Utilisation selon l'une quelconque des revendications 1 à 4, dans laquelle ledit ligand de lipocaline est utilisé en combinaison avec un médicament parasympathomimétique.

Fig. 1

Fig. 2

■ AQP5cRNA 5ng injected oocytes
▨ AQP5cRNA 5ng + lacrimal gland poly(A)RNA 25 ng injected oocytes
☐ H2O injected oocytes

Fig. 3